Europäisches Patentamt

European Patent Office

Office européen des brevets

(11) Publication number: **0 215 567**
**B1**

(12) **EUROPEAN PATENT SPECIFICATION**

(45) Date of publication of patent specification: **04.07.90**

(21) Application number: **86306095.0**

(22) Date of filing: **07.08.86**

(51) Int. Cl.⁵: **C 07 C 11/12,** C 07 C 2/86, C 07 C 1/20

(54) **Production of 2,5-dimethyl-2,4-hexadiene.**

(30) Priority: **07.08.85 JP 173815/85**
**27.12.85 JP 297283/85**
**03.02.86 JP 21697/86**
**30.04.86 JP 101324/86**

(43) Date of publication of application:
**25.03.87 Bulletin 87/13**

(45) Publication of the grant of the patent:
**04.07.90 Bulletin 90/27**

(84) Designated Contracting States:
**CH DE FR GB IT LI NL**

(56) References cited:
**FR-A-2 152 638**
**GB-A- 826 545**
**GB-A- 826 546**
**GB-A- 852 145**
**GB-A- 863 136**
**GB-A- 863 139**
**GB-A- 863 330**

(73) Proprietor: **SUMITOMO CHEMICAL COMPANY,**
**LIMITED**
**Kitahama 4-chome 5-33**
**Chuo-ku Osaka 541 (JP)**

(72) Inventor: **Higashio, Yasuhiko**
**3-11-11, Aobadai**
**Ichihara-shi Chiba 272 (JP)**
Inventor: **Takahashi, Kazuteru**
**Kamiwasedaryo, 135, Iriyamazu**
**Ichihara-shi Chiba 272 (JP)**

(74) Representative: **Sheard, Andrew Gregory et al**
**Kilburn & Strode 30, John Street**
**London WC1N 2DD (GB)**

Courier Press, Leamington Spa, England.

# EP 0 215 567 B1

## Description

This invention relates to an improvement in production of 2,5-dimethyl-2,4-hexadiene by a reaction between isobutylene and/or tert.-butyl alcohol and isobutyl aldehyde.

2,5-dimethyl-2,4-hexadiene is useful as intermediates for agricultural chemicals, insecticides, medicines and other organic synthesis. A method for preparing 2,5-dimethyl-2,4-hexadiene from isobutylene and/or tert.-butyl alcohol with isobutyl aldehyde has been proposed wherein the reactants are allowed to react under pressure in a liquid phase in the presence of mineral acids, sulphonic acids, heteropoly acids or chlorides, sulphates, or phosphates of metals or ammonia (Japanese Patent Kokai 48—34108 corresponding to FR 2152638). The method is not satisfactory, since the yield of 2,5-dimethyl-2,4-hexadiene is as low as less than 40% and is accompanied by a large amount of by-products. With this prior art procedure care must be taken because of corrosion of the apparatus.

G.B. 863330 teaches the production of isoprene by reacting isobutene with formaldehyde at an elevated temperature in the vapour phase in the presence of a catalyst containing 1 to 20% of phosphoric acid deposited on an inert support.

It is clear that this reaction is rather inefficient despite the high activity of formaldehyde. Thus the highest percentage isoprene efficiency or selectivity is only 50% at a formaldehyde conversion of only 32%.

After extensive study to solve the above difficulties, the present inventors find that the reaction between isobutylene and/or tert.-butyl alcohol and isobutyl aldehyde should be conducted in a gaseous phase in the presence of specific solid acid catalysts at a temperature of 150—350°C.

According to the present invention, isobutylene and/or tert.-butyl alcohol is brought into contact with isobutyl aldehyde in a gaseous phase, at a temperature of 150°—350°C, in the presence of a solid acid catalyst selected from a natural clay, a complex oxide, a metal oxide or a synthetic zeolite, the said solid acid catalyst being one whose maximum acidity is stronger than −5.6 in terms of $H_o$-function.

The materials used are isobutylene and/or tert.-butyl alcohol, and isobutyl aldehyde. There is no critical restriction to any materials. So far as isobutylene is concerned, highly pure material may be used as it is, or so-called "spent B—B fraction" may also be used. Isobutylene may be fed in the form of a mixture with tert.-butyl alcohol.

There is no critical limitation in the mixing ratio of isobutylene or tert.-butyl alcohol to isobutyl aldehyde or of a mixture of isobutylene and tert.-butyl alcohol to isobutyl aldehyde. A preferred ratio of isobutylene or tert.-butyl alcohol or a mixture of isobutylene and tert.-butyl alcohol to isobutyl aldehyde is 1—10:1 (in mol.).

The solid catalyst whose maximum acidity is stronger than −5.6 in terms of $H_o$-function may be a natural clay such as acid clay, bentonite, montmorillonite, a complex oxide such as silica-alumina, silica-magnesia, silica-boria, a metal oxide such as niobic acid, or a synthetic zeolite. Preferred catalysts are silica-alumina, acid clay, synthetic zeolite and niobic acid. Particularly preferred is niobic acid, which is known as hydrous niobium oxide. A preparation of niobic acid is disclosed, for example, in Japanese Patent Kokai 60—44039.

The preferred niobic acid catalyst may be used as it is, but, preferably, it is pre-treated with a mineral acid such as sulphuric acid or phosphoric acid. Pre-treatment with mineral acid is effected in such a manner that the niobic acid is dipped in aqueous mineral acid solution and then washed with water. Alternatively, the aqueous mineral acid solution flows through a tower in which niobic acid is packed and then the tower is washed with water. The mineral acid is, preferably, sulphuric acid and phosphoric acid. The concentration of the acid solution varies according to conditions of the pre-treatment, but is usually 0.01—1.0 mol per litre. The amount of the acid solution employed is usually 2—10 times in volume the amount of niobic acid. Niobic acid thus pre-treated is usually calcined at a temperature of 100°—500°C before it is used as a catalyst.

The preferred niobic acid catalyst pre-treated as above is more active and has a higher selectivity for 2,5-dimethyl-2,4-hexadiene than a non-pretreated one. The use of such a pre-treated catalyst can increase the yield of 2,5-dimethyl-2,4-hexadiene.

The reaction is carried out in the gaseous phase and at a temperature of 150°—350°C, preferably 200°—300°C. Reaction at a temperature higher than 350°C is accompanied with a large amount of higher-boiling products such as polymers, while reaction at a temperature lower than 150°C brings about a decrease in reaction speed. Any pressure may be employed as long as the reaction system is kept in the gaseous phase, usually at atmospheric pressure or higher, preferably 2—10 atm (absolute), (2.026 to 10.13 $\times 10^5$ Pascals).

The present reaction is usually carried out in a fixed-bed catalytic reactor wherein the materials are continuously fed in a reactor in which the solid catalyst is packed.

Alternatively, the reaction may be carried out in a fluidized reactor.

The following Examples illustrate the invention.

## Example 1

A tubular reactor made of quartz (inner diameter: 17 mm) was packed with niobic acid catalyst (20 ml, manufacturer: CBMM Co.) and heated to 250°C in an electric furnace. Isobutylene and isobutyl aldehyde

2

were charged therein at rates of 22.4 g/hr and 14.4 g/hr., respectively, and the reaction was effected under atmospheric pressure. Discharged gas was cooled with ice water and light boiling compounds such as unaltered isobutylene were further cooled with dry ice-methanol. The reaction liquid thus obtained was assayed by gas-chromatography to calculate the percentage conversion of isobutyl aldehyde (hereinafter referred to as "conversion") (%), the selectivity for 2,5-dimethyl-2,4-hexadiene (hereinafter referred to as "selectivity") (%) and the yield of 2,5-dimethyl-2,4-hexadiene (hereinafter referred to "yield") (%), all being based on isobutyl aldehyde (mol). The values hereinafter are those at the eighth hour after the start of the reaction, unless otherwise defined.

| Conversion | 89.3% |
| Selectivity | 65.3% |
| Yield | 58.3% |

Examples 2—4

Example 1 was repeated except that the catalysts (20 ml each) shown in Table 1 were used in place of the niobic acid.

Table 1

| Example | Catalyst | Conversion (%) | Selectivity (%) | Yield (%) |
|---------|----------|----------------|-----------------|-----------|
| 2 | Silica-alumina | 83.4 | 58.4 | 48.7 |
| 3 | HY zeolite | 83.5 | 62.6 | 52.8 |
| 4 | acid clay | 79.4 | 52.3 | 41.5 |

Examples 5—8 and Comparison Example 1

Example 1 was repeated except that reaction temperatures and charging rates of isobutylene and isobutyl aldehyde were changed as shown in Table 2. The results are shown in Table 2.

Table 2

| | Temperature (°C) | Isobutylene (g/hr) | Isobutyl aldehyde (g/hr) | Conversion (%) | Selectivity (%) | Yield (%) |
|---|------------------|--------------------|--------------------------|----------------|-----------------|-----------|
| Example 5 | 320 | 44.8 | 28.8 | 97.3 | 58.4 | 56.9 |
| Example 6 | 180 | 11.2 | 7.2 | 81.3 | 64.4 | 52.3 |
| Example 7 | 250 | 44.8 | 14.4 | 93.3 | 68.1 | 63.5 |
| Example 8 | 250 | 11.2 | 14.4 | 80.9 | 58.8 | 47.6 |
| Comparison example 1 | 130 | 11.2 | 7.2 | 32.1 | 40.8 | 13.1 |

3

## Example 9

Example 1 was repeated except that tert.-butyl alcohol (29.6 g/hr) was charged in place of isobutylene (22.4 g/hr).

| | |
|---|---|
| Conversion | 65.3% |
| Selectivity | 65.2% |
| Yield | 42.4% |

## Example 10

A tubular reactor made of SUS (inner diameter = 23 mm) was packed with niobic acid catalyst (20 ml, manufacturer: CBMM Co.) and heated to 250°C in an electric furnace. Isobutylene and isobutyl aldehyde were charged therein at rates of 44.8 g/hr and 14.4 g/hr, respectively, and the reaction was effected under pressure (5 Kg/cm$^2$ or $4.9 \times 10^5$ Nm$^{-2}$ gauge). Discharged gas was depressurized to atmospheric pressure and then cooled with dry ice-methanol. The reaction liquid was assayed by gas-chromatography.

| | |
|---|---|
| Conversion | 95.3% |
| Selectivity | 79.5% |
| Yield | 75.8% |

No formation of diisobutylene was observed, and almost all excess isobutylene was recovered. The reaction was further continued until 300 hours had passed since the reaction initiated;

| | |
|---|---|
| Conversion (300th) | 91.1% |
| Selectivity (300th) | 78.9% |
| Yield (300th) | 72.7% |

Little degradation of catalytic activity was seen.

## Examples 11—14

Example 10 was repeated except that reaction temperatures and pressures were changed as in Table 3. The results are shown in Table 3.

### Table 3

| | Temperature (°C) | Pressure (Kg/cm$^2$ Gauge) | Conversion (%) | Selectivity (%) | Yield (%) |
|---|---|---|---|---|---|
| Example 11 | 300 | 5.0 ($4.9 \times 10^5$ Nm$^{-2}$) | 98.8 | 71,6 | 70.8 |
| Example 12 | 200 | 5.0 ($4.9 \times 10^5$ Nm$^{-2}$) | 81.4 | 68.8 | 56.3 |
| Example 13 | 250 | 10.0 ($9.8 \times 10^5$ Nm$^{-2}$) | 96.6 | 71.9 | 69.5 |
| Example 14 | 250 | 0 | 92.9 | 67.2 | 62.4 |

### Example 15

Example 10 was repeated except that tert.-butyl alcohol (59.2 g/hr) was charged in place of isobutylene (44.8 g/hr).

| | |
|---|---|
| Conversion | 69.6% |
| Selectivity | 68.8% |
| Yield | 48.2% |

### Example 16

Niobic acid catalyst pre-treated with phosphoric acid was prepared as follows.

Niobic acid (100 ml, manufacturer: CBMM Co.) was dipped for two hours at room temperature in 0.1 N/litre aqueous phosphoric acid solution (500 ml). Then, the niobic acid was repeatedly washed (five times) with pure water (1 litre), and calcined for four hours at 350°C.

A tubular reactor made of SUS (inner diameter = 23 mm) was packed with the niobic acid catalyst pretreated above (20 ml) and heated to 250°C in an electric furnace. Isobutylene and isobutyl aldehyde were charged therein at rates of 44.8 g/hr and 14.4 g/hr, respectively, and the reaction was effected under pressure (5 Kg/cm$^2$ or 4.9 × 10$^5$ Nm$^{-5}$ gauge). Discharged gas was de-pressurized to atmospheric pressure and then cooled with dry ice-methanol, before it was assayed by gas-chromatography.

| | |
|---|---|
| Conversion | 97.8% |
| Selectivity | 83.3% |
| Yield | 81.6% |

The reaction was further continued until 300 hours had passed since the reaction initiated:

| | |
|---|---|
| Conversion (300th) | 93.2% |
| Selectivity (300th) | 83.9% |
| Yield (300th) | 78.2% |

Little degradation in catalytic activity and selectivity was seen.

### Examples 17—19

Example 16 was repeated except that reaction temperatures and pressures were changed as in Table 4. The results are shown in Table 4.

### Table 4

| | Temperature (°C) | Pressure (Kg/cm$^2$ Gauge) | Conversion (%) | Selectivity (%) | Yield (%) |
|---|---|---|---|---|---|
| Example 17 | 300 | 5.0 (4.9 × 10$^5$Nm$^{-2}$) | 99.1 | 77.4 | 76.9 |
| Example 18 | 250 | 10.0 (9.8 × 10$^5$Nm$^{-2}$) | 98.5 | 75.5 | 74.4 |
| Example 19 | 250 | 0 | 95.3 | 71.9 | 68.5 |

### Example 20

Example 16 was repeated except that tert.-butyl alcohol (59.2 g/hr) was charged in place of isobutylene (44.8 g/hr).

| | |
|---|---|
| Conversion | 79.4% |
| Selectivity | 80.4% |
| Yield | 63.8% |

Example 21

Niobic acid catalyst pre-treated with sulphuric acid was prepared as follows:

Niobic acid (100 ml, manufacturer: CBMM Co.) was dipped for two hours at room temperature in 0.1 mol/litre aqueous sulphuric acid solution (500 ml). The niobic acid was repeatedly washed (five times) with pure water (1 litre) and calcined for four hours at 350°C.

A tubular reactor made of SUS (inner diameter: 23 mm) was packed with the niobic acid catalyst pre-treated above (20 ml) and heated to 250°C in an electric furnace. Isobutylene and isobutyl aldehyde were charged therein at rates of 448 g/hr and 14.4 g/hr, respectively, and reaction was effected under pressure (5 $Kg/cm^2$ gauge). Discharged gas was depressurized to atmospheric pressure and cooled with dry ice-methanol, before the gas was assayed by gas-chromatography.

| | |
|---|---|
| Conversion | 93.6% |
| Selectivity | 83.3% |
| Yield | 80.5% |

The reaction was further continued until 300 hours had passed since the reaction initiated.

| | |
|---|---|
| Conversion (300th) | 94.1% |
| Selectivity (300th) | 84.1% |
| Yield (300th) | 79.1% |

Little degradation in catalytic activity and selectivity was seen.

Examples 22—24

Example 21 was repeated except that reaction temperatures and pressure were changed as in Table 5. The results are shown in Table 5.

TABLE 5

| | Temperature ($^{\circ}C$) | Pressure ($Kg/cm^2$ Gauge) | Conversion (%) | Selectivity (%) | Yield (%) |
|---|---|---|---|---|---|
| Example 22 | 300 | 5.0<br>$4.9 \times 10^5$<br>Pascals | 98.9 | 79.3 | 78.4 |
| Example 23 | 250 | 10.0<br>$9.8 \times 10^5$<br>Pascals | 97.6 | 79.8 | 77.9 |
| Example 24 | 250 | 0 | 95.2 | 73.8 | 70.3 |

Example 25

Example 21 was repeated except that tert.-butyl alcohol (59.2 g/hr) was charged in place of the isobutylene (44.8 g/hr).

| | |
|---|---|
| Conversion | 80.4% |
| Selectivity | 82.6% |
| Yield | 66.4% |

# EP 0 215 567 B1

## Claims

1. A method for preparing 2,5-dimethyl-2,4-hexadiene from (a) isobutylene and/or tert.butyl alcohol and (b) isobutyl aldehyde characterised in that isobutylene and/or tert.-butyl alcohol are brought into contact with isobutyl aldehyde in a gaseous phase in the presence of a solid acid catalyst selected from a natural clay, a complex oxide, a metal oxide or a synthetic zeolite, the said solid acid catalyst being one whose maximum acidity is stronger than $-5.6$ in terms of $H_o$-function at a temperature of from 150° to 350°C.

2. A method according to Claim 1, wherein the molar ratio of isobutylene or tert.-butyl alcohol or a mixture thereof to isobutyl aldehyde is from 1:1 to 10:1.

3. A method according to Claim 1 or Claim 2 wherein the catalyst is niobic acid.

4. A method according to any one of Claims 1 to 3, wherein the catalyst is niobic acid pre-treated with mineral acid.

5. A method according to Claim 4 wherein the mineral acid is sulphuric acid or phosphoric acid.

6. A method according to Claim 1 or 2, wherein the catalyst is acid clay, silica-alumina, or HY-zeolite.

7. A method according to any one of Claims 1 to 6, wherein the contact is effected under pressure.

8. A method according to any one of Claims 1 to 7, wherein the contact is effected at atmospheric pressure.

9. A method according to any one of Claims 1 to 8, wherein the contact is effected at a temperature of from 200° to 350°C.

## Patentansprüche

1. Verfahren zur Bereitung von 2,5-Dimethyl-2,4-hexadien aus (a) Isobuten und/oder tert.-Butylalkohol und (b) Isobutylaldehyd, dadurch gekennzeichnet, daß Isobuten und/oder tert.-Butylalkohol in einer Gasphase in Anwesenheit eines sauren Festkörperkatalysators mit Isobutylaldehyd in Kontakt gebracht wird, wobei der Katalysator ausgewählt ist aus natürlichem Ton, einem komplexen Oxid, einem Metalloxid oder einem synthetischen Zeolith, und wobei der saure Festkörperkatalysator ein solcher ist, dessen maximale Aciditätstärker als $-5,6$ nach der $H_o$-Aciditätsfunktion bei einer Temperatur von 150° bis 350°C ist.

2. Verfahren nach Anspruch 1, dadurch gekennzeichnet, daß das Molverhältnis von Isobuten oder tert.-Butylalkohol oder einer Mischung von diesen zu Isobutylaldehyd 1:1 bis 10:1 beträgt.

3. Verfahren nach Anspruch 1 oder 2, dadurch gekennzeichnet, daß der Katalysator Niobsäure ist.

4. Verfahren nach einem der Ansprüche 1 bis 3, dadurch gekennzeichnet, daß der Katalysator mit Mineralsäure vorbehandelte Niobsäure ist.

5. Verfahren nach Anspruch 4, bei dem die Mineralsäure Schwefelsäure oder Phosphorsäure ist.

6. Verfahren nach Anspruch 1 oder 2, bei dem der Katalysator saurer Ton, Siliciumoxid-Aluminiumoxid oder HY-Zeolith ist.

7. Verfahren nach einem der Ansprüche 1 bis 6, bei dem der Kontakt unter Druck hergestellt wird.

8. Verfahren nach einem der Ansprüche 1 bis 7, bei dem der Kontakt unter atmosphärischem Druck hergestellt wird.

9. Verfahren nach einem der Ansprüche 1 bis 8, bei dem der Kontakt bei einer Temperatur von 200° bis 350°C hergestellt wird.

## Revendications

1. Procédé de préparation de 2,5-diméthyl-2,4-hexadiène à partir (a) d'isobutylène et/ou d'alcool butylique tertiaire, et (b) d'aldéhyde isobutylique, caractérisé en ce qu'il consiste à mettre l'isobutylène et/ou l'alcool butylique tertiaire en contact avec de l'aldéhyde isobutylique en phase gazeuse, en la présence d'un catalyseur acide solide choisi parmi l'argile naturelle, un oxyde complexe, un oxyde métallique ou un zéolite synthétique, le catalyseur acide solide étant l'un de ceux dont l'activité maximum est supérieure à $-5,6$ exprimée en la fonction $H_o$, à une température de 150° à 350°C.

2. Procédé suivant la revendication 1, dans lequel le rapport molaire de l'isobutylène ou de l'alcool butylique tertiaire ou d'un mélange de ceux-ci à l'aldéhyde isobutylique est compris entre 1:1 et 10:1.

3. Procédé suivant la revendication 1 et la revendication 2, dans lequel le catalyseur est l'acide niobique.

4. Procédé suivant l'une quelconque des revendications 1 à 3, dans lequel le catalyseur est l'acide niobique prétraité par un acide minéral.

5. Procédé suivant la revendication 4, dans lequel l'acide minéral est l'acide sulfurique ou l'acide phosphorique.

6. Procédé suivant la revendication 1 ou 2, dans lequel le catalyseur est une argile acide, de la silicealumine ou un zéolite HY.

7. Procédé suivant l'une quelconque des revendications 1 à 6, qui consiste à effectuer la mise en contact sous pression.

8. Procédé suivant l'une quelconque des revendications 1 à 7, qui consiste à effectuer la mise en contact sous la pression atmosphérique.

9. Procédé suivant l'une quelconque des revendications 1 à 8, qui consiste à effectuer la mise en contact à une température de 200° à 350°C.